(19) Europäisches Patentamt
European Patent Office
Office européen des brevets

(11) Publication number: **0 150 202 B1**

(12) **EUROPEAN PATENT SPECIFICATION**

(45) Date of publication of patent specification: **06.07.88**

(51) Int. Cl.[4]: **C 07 D 401/04**, A 61 K 31/47 // C07C87/62, C07D215/18, C07D215/22, C07D491/044

(21) Application number: **84902808.9**

(22) Date of filing: **18.07.84**

(86) International application number: **PCT/GB84/00252**

(87) International publication number: **WO 85/00602 14.02.85 Gazette 85/04**

(54) **DI/TETRA-HYDROQUINOLINES.**

(30) Priority: **27.07.83 GB 8320184**
**23.12.83 GB 8334415**

(43) Date of publication of application: **07.08.85 Bulletin 85/32**

(45) Publication of the grant of the patent: **06.07.88 Bulletin 88/27**

(84) Designated Contracting States: **CH DE FR GB LI NL**

(56) References cited:
**EP-A-0 076 075**

**Journal of Medicinal Chemistry, vol. 14, no. 11, November 1971, G.C. Wright et al.: "Synthesis and hypotensive properties of new 4-aminoquinolines", pages 1060-1061**

(73) Proprietor: **BEECHAM GROUP PLC**
**Beecham House Great West Road**
**Brentford Middlesex TW8 9BD (GB)**

(72) Inventor: **EVANS, John, Morris**
**"Cata", Old House Lane**
**Roydon, Essex CM19 5DJ (GB)**
Inventor: **STEMP, Geoffrey**
**107 Rundells**
**Harlow, Essex CM18 7HD (GB)**

(74) Representative: **Jones, Pauline et al**
**Beecham Pharmaceuticals Patents & Trade Marks Dept. Great Burgh Yew Tree Bottom Road**
**Epsom Surrey KT18 5XQ (GB)**

**EP 0 150 202 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

The present invention relates to novel compounds having pharmacological activity, to a process for preparing them, to pharmaceutical compositions containing them, and to their use in the treatment of mammals.

EP—A—0076075 describes compounds having blood-pressure lowering activity which are of formula A:

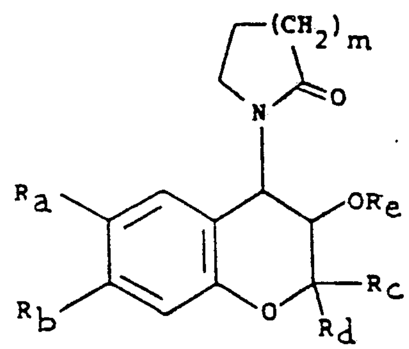

(A)

wherein:

one of $R_a$ and $R_b$ is hydrogen and the other is selected from the class of alkylcarbonyl, alkoxycarbonyl, alkylcarbonyloxy, alkylhydroxymethyl, nitro, cyano, chloro, trifluoromethyl, alkylsulphinyl, alkylsulphonyl, alkoxysulphinyl, alkoxysulphonyl, alkylcarbonylamino, alkoxycarbonylamino or aminosulphinyl, aminosulphonyl or aminocarbonyl, the amino moiety being optionally substituted by one or two alkyl groups, or alkylsulphinylamino, alkylsulphonylamino, alkoxysulphinylamino or alkoxysulphonylamino or ethylenyl terminally substituted by alkylcarbonyl, nitro or cyano, or -C(alkyl)NOH or -C(alkyl)$NNH_2$, the alkyl groups or alkyl moieties of alkyl-containing groups having from 1 to 6 carbon atoms;

one of $R_c$ and $R_d$ is hydrogen or alkyl having from 1 to 4 carbon atoms and the other is alkyl having from 1 to 4 carbon atoms, or $R_c$ and $R_d$ together with the carbon atom to which they are attached are spiroalkyl having from 3 to 6 carbon atoms;

$R_e$ is hydrogen, alkyl having from 1 to 3 carbon atoms or acyl having from 1 to 8 carbon atoms; and m is 1 or 2; the lactam group being trans to the $OR_e$ group.

Accordingly, the present invention provides a compound of formula (I):

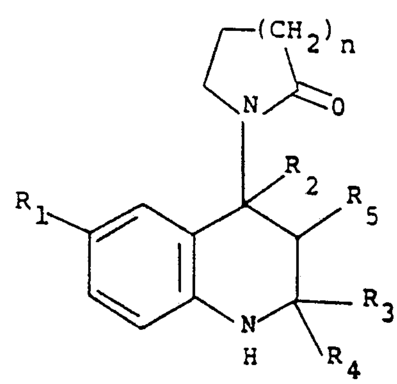

(I)

wherein:

n is 1 or 2;

$R_1$ is chloro, bromo, $C_{1-6}$alkylcarbonyl or cyano;

one of $R_3$ and $R_4$ is hydrogen or $C_{1-4}$ alkyl and the other is $C_{1-4}$ alkyl or $R_3$ and $R_4$ together are $C_{2-5}$ polymethylene;

and either:

$R_5$ is hydroxy, $C_{1-3}$ alkoxy or $C_{1-8}$ acyloxy; and

$R_6$ is hydrogen and the cyclic amide moiety and $R_5$ are *trans*; or $R_5$ and $R_2$ together form a bond, n is often 1.

Suitable values for $R_1$ include chloro, bromo, acetyl, propionyl, *n*-and *iso*-butyryl and cyano. Often $R_1$ is chloro, bromo, acetyl or cyano.

The alkyl groups or alkyl moieties of alkyl-containing groups for $R_1$ are, preferably, methyl or ethyl.

Favourably, $R_3$ and $R_4$ are both alkyl having from 1 to 4 carbon atoms including methyl, ethyl or n-propyl. In particular, they are both methyl or ethyl, preferably both methyl.

Suitable examples for $R_5$ when $C_{1-3}$ alkyloxy include methoxy, ethoxy, *n*- and *iso*-propyoxy, preferably methoxy.

Suitable examples for $R_5$ when acyloxy include carboxylic acyloxy such as acetoxy, propionyloxy and benzoyloxy.

There is a group of compounds within formula (I) of formula (II):

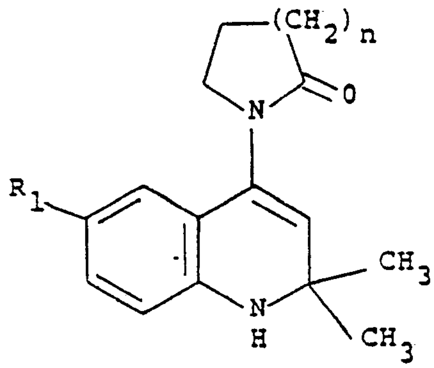

(II)

wherein variables are as defined in formula (I).

Suitable and preferred values for the variables are as described for the corresponding variables under formula (I).

It will be appreciated that there is a favourable sub-group of componds within formula (I) of formula (III):

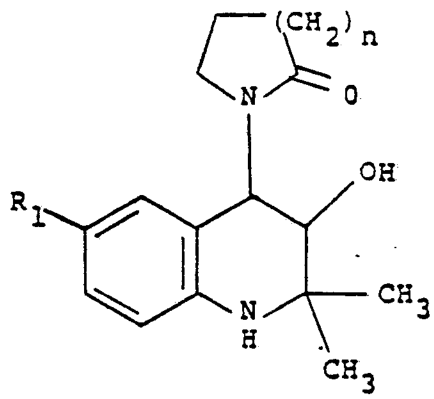

(III)

wherein the variables are as defined in formula (I).

The present invention also provides a process for the preparation of a compound of formula (I) which process comprises reacting a compound of formula (IV):

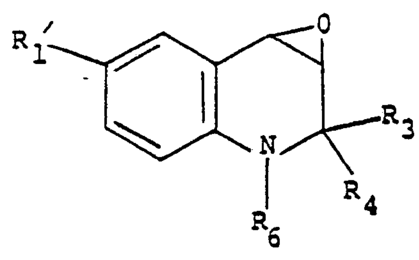

(IV)

with an anion of formula (V):

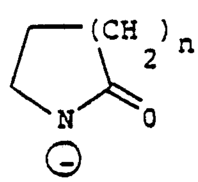

(V)

wherein

$R_1'$ is $R_1$ or a group or atom convertible thereto;

$R_6$ is hydrogen or an N-protecting group;

and the remaining variables are as defined in formula (I), and thereafter if necessary converting $R_1'$ to $R_1$

and/or $R_6$ to hydrogen; optionally $C_{1-3}$ alkylating or $C_{1-8}$ acylating an $R_5$ hydroxy group, or dehydrating a compound of formula (I) wherein $R_5$ is hydroxy to form the compound of formula (I) wherein $R_5$ and $R_2$ together form a bond.

The reaction is suitably carried out in an inert solvent such as dimethylsulphoxide in the presence of a base, such as sodium hydride.

Conversions of $R_1'$ to $R_1$ are generally known in the art of aromatic chemistry. For example, $R_1'$ when halo, preferably bromo, may be converted to an $R_1$ cyano group by heating with copper (I) cyanide in an inert solvent, such as N-methylpyrrolidone. This process is preferred when $R_1$ is cyano in formula (I)

Suitable N-protecting groups $R_6$ include acyl groups such as $C_{2-5}$ alkanoyl for example acetyl and propionyl; benzoyl, phthalolyl or readily hydrogenolysable groups such as benzyl or benzyloxycarbonyl.

Preferably an $R_6$ protecting group is $C_{2-5}$ alkanoyl such as acetyl or propionyl. Such groups may be removed by conventional hydrolysis.

An $R_6$ acetyl group is particularly easily removed and may therefore under certain conditions be converted to an $R_6$ hydrogen by chromatographic methods, such as on a silica gel column.

Alkylation of $R_5$ hydroxy may be carried out using $R_51$ $L_2$ wherein $L_2$ is a leaving group and $R_5'$ is $C_{1-3}$ alkyl. $L_2$ is a group readily displaceable by a nucleophile, preferably halo such as iodo. The reaction is carried out in an inert solvent, such as toluene, in the presence of a base, such as potassium *t*-butoxide.

Acylation of an $R_5$ hydroxy group may be carried out using a carboxylic acid or a derivative thereof, such as an anhydride, the reaction being carried out in a non-hydroxylic solvent in the presence of a condensation promoting agent, such as dicyclohexyl-carbodiimide.

It will be appreciated that the above acylations/alkylations at $R_5$ may affect the substitution at the hydroquinoline nitrogen atom and appropriate modifications of reaction conditions and/or appropriate protection of should be carried out during the reaction. It will also be appreciated that such conversions may take place in any desired or necessary order.

Compounds of formula (IV) may be prepared by reacting a compound of formula (VI):

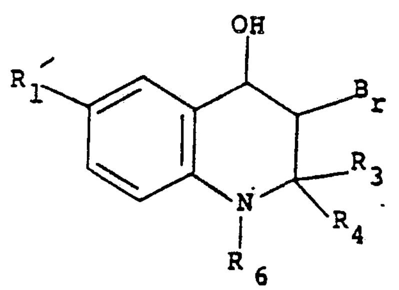

(VI)

wherein the variables are as hereinbefore defined; with a base, such as potassium hydroxide, in ether or aqueous dioxan.

Compounds of the formula (VI) may be prepared in accordance with known processes, for example as described in the following Scheme:

Scheme

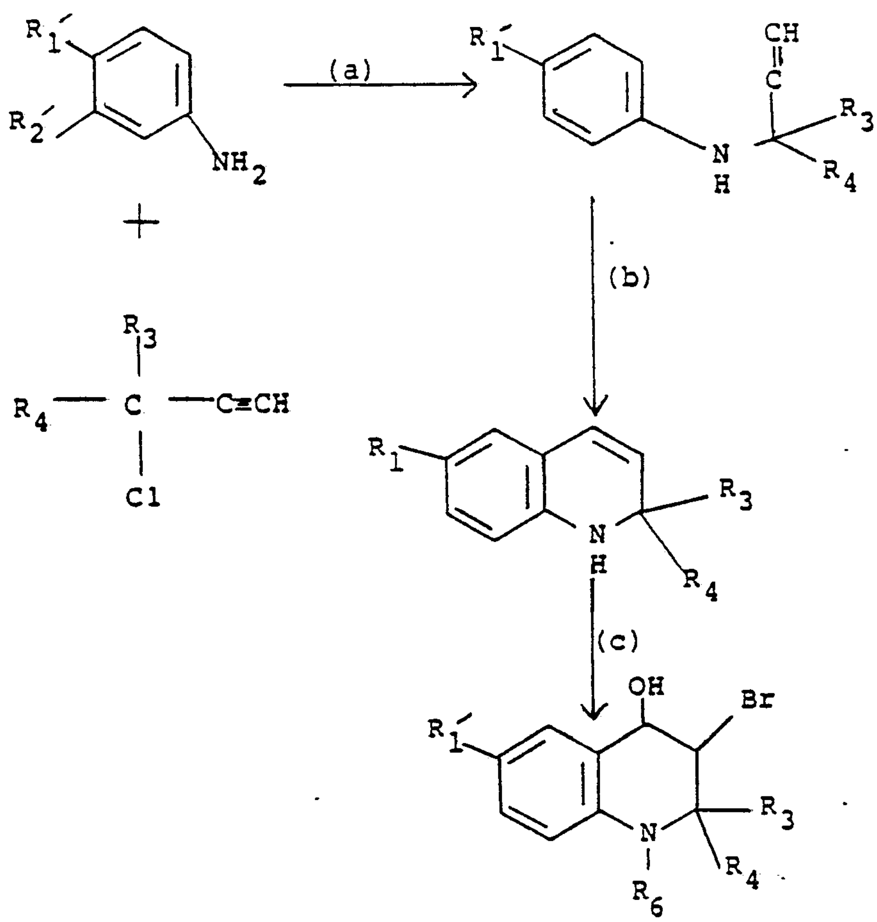

a) Room temperature; triethylamine; copper (I) chloride, copper bronze; water; ether.

b) Heat to 80°C; copper (I) chloride in doixan under $N_2$.

c) Optional N-protection e.g. acetylation with acetyl chloride in N,N-dimethylaniline in dichloromethane; then N-bromo succinimide/dimethylsulphoxide/water.

The above process can produce mixtures of compounds during reaction b) owing to the two sites available for ring formation. It is therefore advisable to remove any of the undesired compound by, for example, chromatography, before reaction c).

Instead of carrying out the conversion of $R_1'$ when a group or atom convertible into $R_1$ after reacting a compound of formula (IV) with an anion of formula (V), it is greatly preferred that any such conversions are carried out at an earlier stage, preferably on the dihydroquinoline produced after reaction b) above. In other words, it is preferred that, for the process of the invention $R_1'$ is $R_1$, except when $R_1$ is cyano as hereinbefore described.

As mentioned previously, the compounds of formula (I) exist in optionally active forms, and the processes of the present invention produce mixtures of such forms. The individual isomers may be separated one from another by chromatography using a chiral phase. Alternatively, as asymmetric synthesis would offer a route to the individual form.

The intermediates of formulae (IV) and (VI) are novel and form an aspect of the present invention.

As mentioned previously, the compounds of formula (I) have been found to have blood-pressure lowering activity. They are therefore useful in the treatment of hypertension.

The present invention accordingly provides a pharmaceutical composition which comprises a compound of formula (I) and a pharmaceutically acceptable carrier. In particular, the present invention provides an anti-hypertensive pharmaceutical composition which comprises an anti-hypertensive effective amount of a compound of formula (I) and a pharmaceutically acceptable carrier.

The compositions are preferably adapted for oral administration. However, they may be adapted for other modes of administration, for example parenteral administration for patients suffering from heart failure.

In order to obtain consistency of administration it is preferred that a composition of the invention is in the form of a unit-dose. Suitable unit dose forms include tablets, capsules and powders in sachets or vials. Such unit dose forms may contain from 1 to 100 mg of a compound of the invention and more usually from

2 to 50 mg, for example 5 to 25 mg as 6, 10, 15 or 20 mg. Such compositions may be administered from 1 to 6 times a day, more usually from 2 to 4 times a day, in a manner such that the daily dose is from 5 to 200 mg for a 70 kg human adult and more particularly from 10 to 100 mg.

The compositions of the invention may be formulated with conventional excipients, such as a filler, a disintegrating agent, a binder, a lubricant, a flavouring agent. They are formulated in conventional manner, for example in a manner similar to that used for known anti-hypertensive agents, diuretics and β-blocking agents.

The present invention further provides a compound of formula (I) for use in the treatment of hypertension.

The present invention yet further provides a compound for use in the treatment of hypertension in mammals including man, which treatment comprises administering to the suffering mammal an anti-hypertensive effective amount of a compound of formula (I) or a pharmaceutical composition.

The following examples illustrate the preparation of compounds of formula (I);

The following descriptions relate to the preparation of intermediates.

Description 1

4-Chloro-N-(1,1-dimethyl-2-propynyl)-aniline

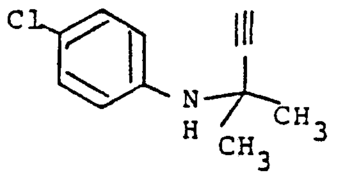

(D1)

3-Chloro-3-methyl-1-butyne (24.0 g) was added dropwise to a vigorously stirred mixture of 4-chloroaniline (25.6 g), triethylamine (20.5 g), copper (1) chloride (0.4 g), copper bronze (0.4 g), water (20 ml) and ether (100 ml), while maintaining a temperature of 15—22°C. After stirring for 2.5 hours at room temperature, the mixture was poured into ether (200 ml) and water (100 ml). The ethereal layer was separated, and washed with water, and then brine before drying over potassium carbonate and potassium hydroxide. Removal of drying agent and solvent gave a brown oil which was distilled to give the title compound (30.0 g) which had b.pt. 68—74°C at 0.06 mm Hg (G. F. Hennion and R. S. Hanzel, J.A.C.S., *82*, 4908, (1960) quote b.pt. 98—99°C at 0.45 mm Hg);

Nmr ($CDCl_3$)
1.55 (s, 6H)
2.30 (2, 1H)
3.45 (N<u>H</u>),
6.80 (d, J=9, 2H)
7.20 (d, J=9, 2H)

$M^+$ 193.660 ($C_{11}H_{12}ClN$ requires $M^+$ 193.0658).

Description 2

6-Chloro-2,2-dimethyl-1,2-dihydroquinoline

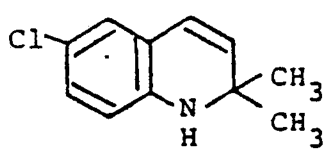

(D2)

A mixture of 4-chloro-N-(2,2-dimethyl-2-propynyl)-aniline (32.5 g) and copper (1) chloride (17.0 g) in dioxan (200 ml) was stirred at 80°C for 2 hours, under nitrogen. The mixture was cooled, diluted with ether, and filtered. The organic phase was washed with water, then brine before drying over magnesium sulphate. Removal of drying agent and solvent, followed by distillation of the residue, gave the dihydroquinoline (18.0 g) which had b.t. 85—100°C at 0.06 mm Hg;

Nmr ($CDCl_3$)
1.25 (s, 6H)
3.45 (N<u>H</u>)
5.35 (d, J=9, 1H)
6.10 (d, J=9, 1H)
6.20 (d, J=10, 1H);
6.70 (m, 2H)

$M^+$ 193.0658 ($C_{11}H_{12}NCl$ requires $M^+$ 193.0658).

Description 3

1-Acetyl-6-chloro-2,2-dimethyl-1,2-dihydroquinine

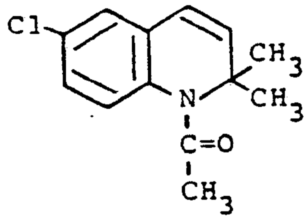

(D3)

Acetyl chloride (12 ml) was added dropwise to a stirred solution of 6-chloro-2,2-dimethyl-1,2-dihydroquinoline (16.0 g) and N,N-dimethylaniline (24 ml) in dichloromethane (200 ml) at 0°C. The solution was stirred for a further 24 hours at room temperature, then poured into water and the organic layer separated, and washed successively with 1N hydrochloric acid, 5% sodium bicarbonate solution, water, brine and then dried over sodium sulphate. Removal of drying agent and solvent gave the crude N-acetyl derivative as a gum (18.9 g) having

Nmr ($CDCl_3$)

1.50 (s, 6H)
2.10 (s, 3H)
5.60 (d, J=10, 1H)
6.15 (d, J=10, 1H)
6.60 (d, J=8, 1H)
6.90 (m, 2H)

$M^+$ 235.0766 ($C_{13}H_{14}ClNo$ requires $M^+$ 235.0764).

Description 4

1-Acetyl-3-bromo-6-chloro-2,2-dimethyl-1,2,3,4-tetrahydroquinolin-4-ol

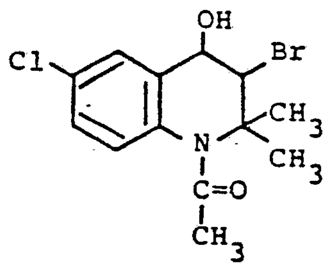

(D4)

N-Bromosuccinimide (16.0 g) was added to a vigorously stirred solution of 1-acetyl-6-chloro-2,2-dimethyl-1,2-dihydroquinoline (18.9 g) in dimethyl sulphoxide (150 ml) containing water (15 ml). The mixture was stirred for 1 hour at room temperature, then diluted with water and extracted into ethyl acetate. The organic extracts were washed with water and brine, then dried over sodium sulphate. Removal of drying agent and solvent gave the bromohydrin as a gum (26.5 g) which had

Nmr ($CDCl_3$)

1.70 (s, 6H)
2.10 (s, 3H)
3.30 (O<u>H</u>)
3.80 (d, J=9, 1H)
4.70 (d, J=9, 1H)
6.75 (d, J=8, 1H)
7.10 (dd, J=8, 2, 1H)
7.45 (d, J=2, 1H)

$M^+$ 330.9972 ($C_{13}H_{15}BrClNO_2$ requires $M^+$ 330.9975).

Description 5

1-Acetyl-6-chloro-2,2-dimethyl-3,4-epoxy-1,2,3,4-tetrahydroquinoline

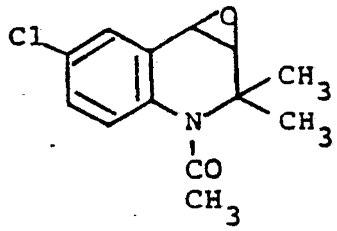

(D5)

A mixture of 1-acetyl-3-bromo-6-chloro-2,2-dimethyl-1,2,3,4-tetrahydroquinolin-4-ol (26.5 g) and potassium hydroxide pellets (25.0 g) in dry ether (500 ml) was vigorously stirred at room temperature for 48 hours. The solution was filtered and evaporated *in vacuo* to give the epoxide (18.5 g) as a gum having

Nmr ($CDCl_3$)
1.20 (s, 3H)
1.85 (s, 3H)
2.10 )s, 3H)
3.35 (d, J=4, 1H)
3.75 (d, J=4, 1H)
6.65 (d, J=9, 1H)
7.15 (m, 2H)

Example 1

Trans-2,2-dimethyl-6-chloro-4-(2-oxo-pyrrolidinyl)-1,2,3,4-tetrahydroquinolin-3-ol

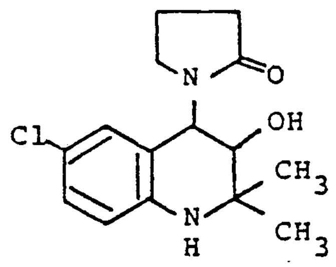

(1)

To a suspension of sodium hydride (450 mg of 80% dispersion in oil) in dry dimethylsulphoxide (20 ml) was added 2-pyrrolidinone (1.2 g) in dimethylsulphoxide (20 ml) and the mixture was then stirred at room temperature, under nitrogen, for 1 hour. A solution of 1-acetyl 6-chloro-2,2-dimethyl-3,4-epoxy-1,2,3,4-tetrahydroquinoline (2.5 g) in dimethyl sulphoxide was then added dropwise, and the solution stirred for 24 hours at room temperature. The mixture was then poured into water and extracted into ethyl acetate. The combined organic layers were washed with water, then brine, and dried over sodium sulphate. Removal of solvent and drying agent, followed by chromatography of the residue on a silica gel column eluted with mixtures of chloroform and methanol, gave the title compound as a yellow foam (1.1 g) which recrystallised from 60—80 petrol-ethyl acetate (9:1) as colourless microprisms (0.24 g) having m.pt 82—85°C;

Nmr ($CDCl_3$)
1.20 (s, 3H)
1.30 (s, 3H)
2.10 (m, 2H)
2.55 (m, 2H)
2.80 (O<u>H</u>)
3.20 (m, 2H)
3.65 (d, J=10, 1H)
3.75 (N<u>H</u>)
5.25 (d, J=10, 1H)
6.45 (d, J=8, 1H)
6.95 (m, 2H)

$M^+$ 294.1134 ($C_{15}H_{19}N_2O_2Cl$ requires $M^+$ 294.1134).

Example 2

6-Chloro-2,2-dimethyl-4-(2-oxo-pyrrolidinyl)-1,2-dihydroquinoline

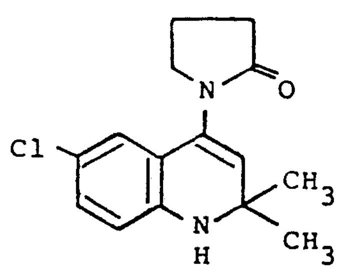

(2)

From the reaction between 2-pyrrolidinone and 6-chloro-2,2-dimethyl-3,4-epoxy-1,2,3,4-tetrahydroquinoline described in Example 1, investigation of the earlier chromatographic fractions gave the title

compound as a yellow foam, which recrystallised from 60—80 petrol-ethyl acetate (9:1) as yellow plates (0.8 g) having m.pt. 158—9°C;

Nmr ($CDCl_3$)

1.35 (s, 6H)
2.20 (m, 2H)
2.50 (m, 2H)
3.60 (t, J=6, 2H)
3.75 (N<u>H</u>)
5.50 (s, 1H)
6.40 (d, J=8, 1H)
6.75 (d, J=2, 1H)
6.95 (dd, J=8, 2, 1H)

$M^+$ 276.1008 ($C_{15}H_{17}N_2OCl$ requires $M^+$ 276.1010); *Anal.* Found: C, 65.09; H, 6.18; N, 10.14; $C_{15}H_{17}N_2OCl$ requires C, 65.10; H, 6.19; N, 10.12.

Example 3

Trans-2,2-dimethyl-6-bromo-4-(2-oxo-pyrrolidinyl)-1,2,3,4-tetrahydroquinolin-3-ol

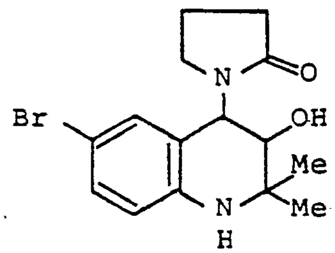

(3)

By an analogous procedure to that found in Example 1, reaction between 2-pyrrolidinone (3.4 g) and 1-acetyl-6-bromo-2,2-dimethyl-3,4-epoxy-1,2,3,4-tetrahydroquinoline (12.0 g) using sodium hydride (1.5 g) as base in dimethylsulphoxide (70 ml) gave the title compound, after chromatography, as an off-white foam (0.7 g) having m.pt. 93—102°C.

Nmr ($CDCl_3$)

1.20 (s, 3H)
1.35 (s, 3H)
2.20 (m, 2H)
2.60 (m, 2H)
3.20 (m, 2H)
3.60 (O<u>H</u>, N<u>H</u>)
3.70 (d, J=10, 1H)
5.20 (d, J=10, 1H)
6.40 (d, J=8, 1H)
7.10 (m, 2H)

$M^+$ 338.0639 ($C_{15}H_{10}N_2O_2Br$ requires $M^+$ 338.0630).

Example 4

Trans-2,2-dimethyl-6-cyano-4-(2-oxo-pyrrolidinyl)-1,2,3,4-tetrahydroquinolin-3-ol

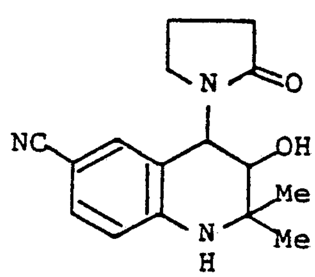

(4)

A solution of *trans*-2,2-dimethyl-6-bromo-4-(2-oxo-pyrrolidinyl)-1,2,3,4-tetrahydroquinolin-3-ol (0.37 g), and copper (1) cyanide (0.2 g) in N-methylpyrrolidone (10 ml) was heated under reflux for 4 h. After cooling, the mixture was poured into 10% aqueous ethylenediamine (50 ml) and extracted into ethyl acetate. The combined extracts were washed with water, and dried over magnesium sulphate. Removal of solvent and

drying agent, followed by crystallisation of the residue from ethyl acetate gave the title compound as off-white microcrystals (76 mg) having m.pt. 297—298°C;

Nmr (DMDSO-$d_6$)

1.10 (s, 3H)
1.30 (s, 3H)
2.00 (m, 2H)
2.35 (m, 2H)
3.00—3.75 (m, hidden by $H_2O$, 3H)
4.90 (d, J=10, 1H)
5.35 (d, J=4, 1H, exchangeable with $D_2O$)
6.65 (d, J=8, 1H)
6.80 (s, 1H, exchangeable with $D_2O$)
7.05 (m, 1H)
7.36 (dd, J=8, 2, 1H)

$M^+$ 285.1481 ($C_{16}H_{19}N_3O_2$ requires $M^+$ 285.1477); *Anal.* Found: 67.35; H, 6.74; N, 14.64; $C_{16}H_{19}N_3O_2$ requires C, 67.35; H, 6.71; N, 14.73.

Pharmacological Data

Systolic blood pressures were recorded by a modification of the tail cuff method described by I. M. Claxton, M. G. Palfreyman, R. H. Poyser, R. L. Whiting, European Journal of Pharmacology, *37,* 179 (1976). W+W BP recorder, model 8005, was used to display pulses prior to all measurements rats were placed in a heated environment (33.5±0.5°C) before transfer to a restraining cage. Each determination of blood pressure was the mean of at least 6 readings. Spontaneously hypertensive rats (ages 12—18 weeks) with systolic blood pressures >170 mmHg were considered hypertensive.

| Compound of Example 4 | Time Post Dose Hours | % Change in Systolic Blood Blood | % Change in Heart Rate |
|---|---|---|---|
| 6 rats Dose 3 mg/kg | 1 | − 2 ± 6 | − 4 ± 4 |
| p.o. | 2 | − 8 ± 6 | −11 ± 2 |
| Initial Blood Pressure | 4 | −26 ± 5 | − 6 ± 6 |
| 209 ± 6 mmHg | 6 | −19 ± 4 | − 7 ± 5 |
| Initial Heart Rate 465 ± 16 beats/min | 24 | − 3 ± 9 | − 5 ± 6 |

| Compound of Example 1 | Time Post Dose Hours | % Change in Syhstolic Blood Blood | % Change in Heart Rate |
|---|---|---|---|
| 6 rats Dose 10 mg/kg | 1 | −25 ± 1 | 3 ± 2 |
| p.o. | 2* | −18 ± 3 | 1 ± 3 |
| Initial Blood Pressure | 4 | −16 ± 4 | −6 ± 2 |
| 246 ± 8 mmHg | 6 | −10 ± 4 | −4 ± 3 |
| Initial Heart Rate | 491 ± 14 beats/min | | |

*At 2 hours 1 rat had no measurable pulse.

**Claims**

1. A compound of formula (I):

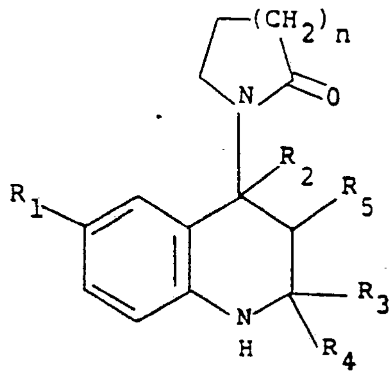

(I)

wherein:

n is 1 or 2;

$R_1$ is chloro, bromo, $C_{1-6}$ alkylcarbonyl or cyano;

one of $R_3$ and $R_4$ is hydrogen or $C_{1-4}$ alkyl and the other is $C_{1-4}$ alkyl or $R_3$ and $R_4$ together are $C_{2-5}$ polymethylene;

and ether;

$R_5$ is hydroxy, $C_{1-3}$ alkoxy or $C_{1-8}$ acyloxy; and

$R_2$ is hydrogen and the cyclic amide moiety and $R_5$ are *trans*; or $R_5$ and $R_2$ together form a bond.

2. A compound according to claim 1 wherein $R_3$ and $R_4$ are both methyl.

3. A compound according to claim 1 or 2 of formula (III):

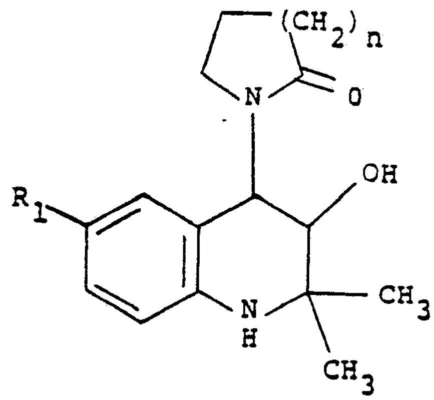

(III)

wherein $R_1$ and n are as defined in formula (I).

4. A compound according to any one of claims 1, 2 or 3 wherein n is 1.

5. A compound according to any one of claims 1 to 4 wherein $R_1$ is chloro, bromo, acetyl or cyano.

6. Trans-2,2-dimethyl-6-chloro-4-(2-oxo-pyrrolidinyl)-1,2,3,4-tetrahydroquinolin-3-ol,
6-chloro-2,2-dimethyl-4-(2-oxo-pyrrolidinyl)-1,2-dihydroquinoline,
trans-2,2-dimethyl-6-bromo-4-(2-oxo-pyrrolidonyl)-1,2,3,4-tetrahydroquinolin-3-ol or
trans-2,2-dimethyl-6-cyano-4-(2-oxo-pyrrolidinyl)-1,2,3,4-tetrahydroquinolin-3-ol.

7. A process for the preparation of a compound according to any one of claims 1 to 5 which process comprises reacting a compound of formula (IV):

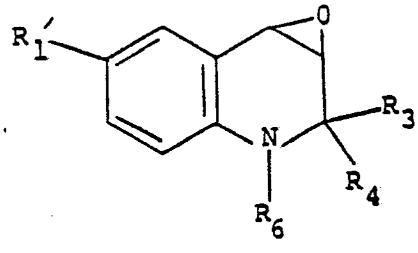

(IV)

with an anion of formula (V):

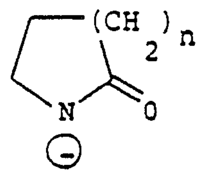

(V)

wherein $R_1'$ is $R_1$ or a group or atom convertible thereo;

$R_6$ is hydrogen or an N-protecting group;

and the remaining variables are as defined in claim 1 and thereafter if necessary converting $R_1'$ to $R_1$ and/or $R_6$ to hydrogen; optionally $C_{1-3}$ alkylating or $C_{1-8}$ acylating an $R_5$ hydroxy group, or dehydrating a compound of formula (I) wherein $R_5$ is hydroxy to form the compound of formula (I) wherein $R_5$ and $R_2$ together form a bond.

8. A pharmaceutical composition comprising a compound according to any one of claims 1 to 6 and a pharmaceutically acceptable carrier.

9. A compound according to any one of Claims 1 to 6 for use as an active therapeutic substance.

10. A compound according to any one of Claims 1 to 6, for use in the treatment of hypertension.

11. The use of a compound, according to any one of Claims 1 to 6, in the preparation of a medicament for treatment of hypertension.

**Patentansprüche**

1. Eine Verbindung der Formel (I):

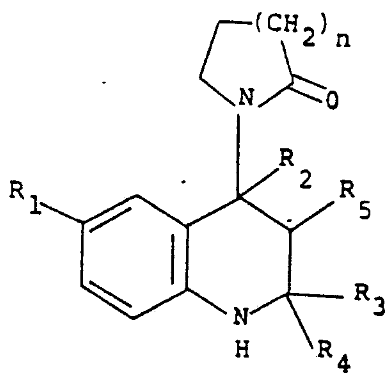

(I)

in welcher

n den Wert 1 oder 2 hat;

$R_1$ Chlor, Brom, $C_{2-6}$-Alkylcarbonyl oder Cyano bedeutet; eine der Gruppen $R_3$ und $R_4$ Wasserstoff oder $C_{1-4}$-Alkyl ist und die andere Gruppe $C_{1-4}$-Alkyl bedeutet oder $R_3$ und $R_4$ zusammen ein $C_{2-5}$-Polymethylen darstellen;

und entweder

$R_5$ Hydroxy, $C_{1-3}$-Alkoxy oder $C_{1-8}$-Acyloxy ist; und

$R_2$ Wasserstoff ist und die zyklische Amidgruppe und

$R_5$ sich in der trans-Stellung befinden; oder $R_5$ und $R_2$ zusammen eine Bindung bilden.

2. Eine Verbindung nach Anspruch 1, in welcher $R_3$ und $R_4$ jeweils eine Methylgruppe sind.

3. Eine Verbindung nach Anspruch 1 oder 2 der Formel (III):

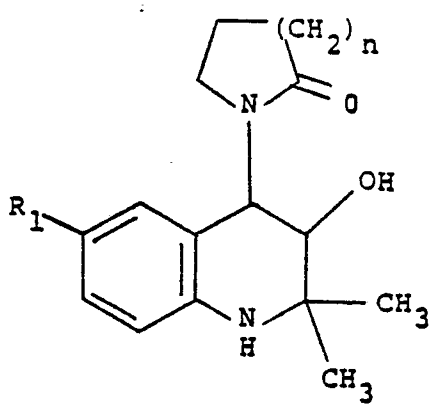

(III)

in welcher $R_1$ und n wie in bezug auf Formel (I) definiert sind.

4. Eine Verbindung nach einem der Ansprüche 1, 2 oder 3, in welcher n den Wert 1 hat.

5. Eine Verbindung nach einem der Ansprüche 1 bis 4, in welcher $R_1$ eine Chlor-, Brom-, Acetyl-, oder Cyanogruppe ist.

6. trans-2,2-Dimethyl-6-chlor-4-(2-oxo-pyrrolidinyl)-1,2,3,4-tetrahydrochinolin-3-ol,
6-Chlor-2,2-dimethyl-4-(2-oxo-pyrrolidinyl)-1,2-dihydrochinolin,
trans-2,2-Dimethyl-6-brom-4-(2-oxo-pyrrolidinyl)-1,2,3,4-tetrahydrochinolin-3-ol oder
trans-2,2-Dimethyl-6-cyano-4-(2-oxo-pyrrolidinyl)-1,2,3,4-tetrahydrochinolin-3-ol.

7. Ein Verfahren zur Herstellung einer Verbindung nach einem der Ansprüche 1 bis 5, welches Verfahren die Umsetzung einer Verbindung der Formel (IV):

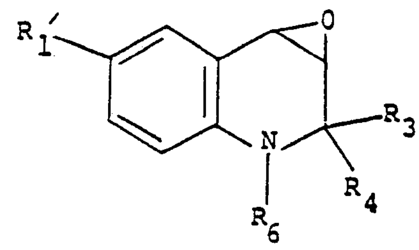

(IV)

mit einem Anion der Formel (V):

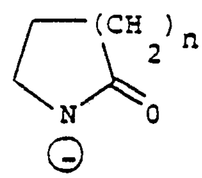

(V)

in welcher $R_1'$ $R_1$ oder eine in diese Gruppe umwandelbare Gruppe oder ein umwandelbares Atom ist; $R_6$ Wasserstoff oder eine N-Schutzgruppe ist; und die verbleibenden Variablen wie in Anspruch 1 definiert sind, und anschließend, falls notwendig, die Umwandlung der Gruppe $R_1'$ in $R_1$ und/oder der Gruppe $R_6$ in Wasserstoff; gegebenenfalls $C_{1-3}$-Alkylieren oder $C_{1-8}$-Acylieren einer $R_5$-Hydroxygruppe, oder das Dehydrieren einer Verbindung der Formel (I), in welcher $R_5$ Hydroxy bedeutet, zu einer Verbindung der Formel (I), in welcher $R_5$ und $R_2$ zusammen eine Bindung bilden, umfaßt.

8. Eine pharmazeutische Zusammensetzung umfassend eine Verbindung gemäß einem der Ansprüche 1 bis 6 und einen pharmazeutisch verträglichen Träger.

9. Eine Verbindung nach einem der Ansprüche 1 bis 6 zur Verwendung als aktive therapeutische Substanz.

10. Eine Verbindung nach einem der Ansprüche 1 bis 6, zur Verwendung bei der Behandlung von Bluthochdruck.

11. Die Verwendung einer Verbindung nach einem der Ansprüche 1 bis 6, bei der Herstellung eines Medikaments zur Behandlung von Bluthochdruck.

**Revendications**

1. Composé caractérisé par la formule (I):

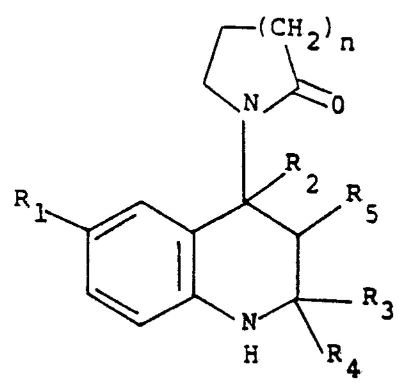

(I)

dans laquelle
n est 1 ou 2;
$R_1$ est un atome de chlore ou de brome, un groupe alcoyl-($C_{1-6}$)carbonyle ou cyano; l'un des $R_3$ et $R_4$

est un atome d'hydrogène ou un groupe alcoyle en $C_{1-4}$ et l'autre est un groupe alcoyle en $C_{1-4}$ ou bien $R_3$ et $R_4$ sont ensemble un groupe méthylène en $C_{1-4}$; et soit

$R_5$ est un groupe hydroxy, alcoxy en $C_{1-3}$ ou acyloxy en $C_{1-8}$; et

$R_2$ est un atome d'hydrogène et la partie amide cyclique et $R_5$ sont *trans*; ou $R_4$ et $R_2$ forment ensemble une liaison.

2. Composé suivant la revendication 1, caractérisé en ce que $R_3$ et $R_4$ sont tous deux un groupe méthyle.

3. Composé suivant la revendication 1 ou 2, caractérisé par la formule (III):

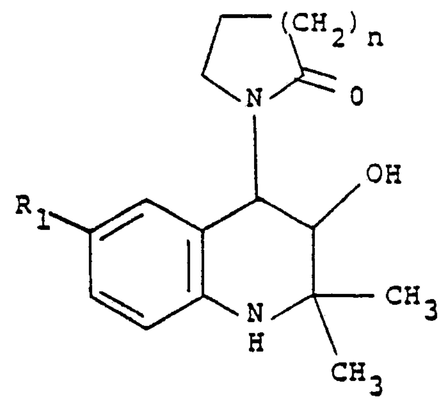

(III)

dans laquelle $R_1$ et n sont tels que définis pour la formule (I).

4. Composé suivant l'une quelconque des revendications 1, 2 ou 3, caractérisé en ce que n est égal à 1.

5. Composé suivant l'une quelconque des revendications 1 à 4, caractérisé en ce que $R_1$ est un atome de chlore, de brome, un groupe acétyle ou cyano.

6. Composé suivant la revendication 1, caractérisé en ce qu'il s'agit du
trans-2,2-diméthyl-6-chloro-4-(2-oxo-pyrrolidinyl)-1,2,3,4-tétrahydroquinoléin-3-ol,
de la 6-chloro-2,2-diméthyl-4-(2-oxo-pyrrolidinyl)-1,2-dihydroquinoléine,
du trans 2,2-diméthyl-6-bromo-4-(2-oxo-pyrrolidinyl)-1,2,3,4-tétrahydroquinoléin-3-ol, ou
du trans-2,2-diméthyl-6-cyano-4-(2-oxo-pyrrolidinyl)-12,3,4-tétrahydroquinoléin-3-ol.

7. Procédé de préparation d'un composé suivant l'une quelconque des revendication 1 à 5, caractérisé en ce qu'il comprend la réaction d'un composé de formule (IV):

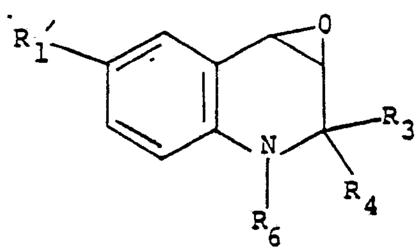

(IV)

avec un anion de formule (V):

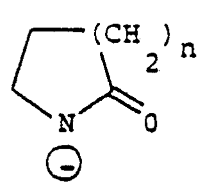

(V)

dans laquelle $R_1'$ est $R_1$ ou un groupe ou un atome pouvant être converti en celui-ci;

$R_6$ est un atome d'hydrogène ou un groupe N-protecteur;

et les variables restantes sont telles que définies dans la revendication 1 et ensuite, si cela est nécessaire, la conversion de $R_1'$ en $R_1$ et/ou de $R_6$ en atome d'hydrogène; une éventuelle alcoylation en $C_{1-3}$ ou acylation en $C_{1-8}$ du groupe $R_5$ hydroxy, ou la déshydratation d'un composé de formule (I) dans laquelle $R_5$ est un groupe hydroxy pour former le composé de formule (I) dans laquelle $R_5$ et $R_2$ forment ensemble une liaison.

8. Composition pharmaceutique caractérisée en ce qu'elle comprend un composé suivant l'une quelconque des revendications 1 à 6 et un support acceptable du point de vue pharmaceutique.

9. Composé suivant l'une quelconque des revendication 1 à 6, caractérisé en ce qu'il est utile en tant que substance thérapeutique active.

10. Composé suivant l'une quelconque des revendications 1 à 6, caractérisé en ce qu'il est utile dans le traitement de l'hypertension.

11. Utilisation d'un composé suivant l'une quelconque des revendications 1 à 6, caractérisée en ce qu'elle correspond à la préparation d'un médicament pour le traitement de l'hypertension.